# EUROPÄISCHE PATENTANMELDUNG

(11) **EP 2 255 958 A2**
(43) Veröffentlichungstag der Anmeldung: **01.12.2010**
(21) Anmeldenummer: 10164522.4
(22) Anmeldetag: 31.05.2010
(51) Int. Cl.: B29C 70/64, C04B 41/91, C12N 11/00, G01N 33/543, G01N 33/545

(54) **Formkörper mit eingebetteten Kopplungspartikeln für Biomoleküle**

(30) Priorität: 29.05.2009 DE 102009026622
(71) Anmelder: Fraunhofer-Gesellschaft zur Förderung der Angewandten Forschung e.V., 80686 München (DE)
(72) Erfinder: Salk, Natalie, 27798 Hude (DE); Grunwald, Ingo, 28205 Bremen (DE)
(74) Vertreter: Eisenführ, Speiser & Partner

(57) **Zusammenfassung**

Die Erfindung betrifft einen Formkörper, umfassend eine Matrix aus einem Material, ausgewählt aus der Gruppe bestehend aus Metall, Keramik und polymerem Kunststoff, und in die Matrix eingebettete Kopplungspartikel, wobei ein Teil der Oberfläche des Formkörpers in einer geometrischen Form oder einem regelmäßigen Muster und/oder eine Fläche des Formkörpers vollständig oder die gesamte Oberfläche des Formkörpers mechanisch bearbeitet ist.

## Beschreibung

Die Erfindung betrifft einen Formkörper, umfassend eine Matrix aus einem Material, ausgewählt aus der Gruppe bestehend aus Metall, Keramik und polymerem Kunststoff und in die Matrix eingebettete Kopplungspartikel, wobei ein Teil der Oberfläche des Formkörpers in eine geometrische Form oder einem regelmäßigen Muster und/oder eine Fläche des Formkörpers vollständig oder die gesamte Oberfläche des Formkörpers mechanisch bearbeitet ist.

Insbesondere betrifft die Erfindung einen Formkörper, umfassend eine Polymerkunststoffmatrix und darin eingebettete Kopplungspartikel, wobei der Formkörper mechanisch bearbeitet ist, so dass ein Teil der Partikel wenigstens von der Kunststoffmatrix bzw. der umgebenen Metall- oder Keramikmatrix unbedeckt ist, und wobei ein Teil der Oberfläche des Formkörpers in einer geometrischen Form oder einem regelmäßigen Muster und/oder eine Fläche des Formkörpers vollständig oder die gesamte Oberfläche des Formkörpers mechanisch bearbeitet ist.

Die Erfindung betrifft ferner ein Verfahren zur Herstellung eines solchen Formkörpers und die Verwendung eines solchen Formkörpers als Sensor, Biochip, zu Diagnostikzwecken für immunologische und zellbiologische Nachweisverfahren, als Bioreaktor oder Bestandteil eines Bioreaktors, für Labor-auf-dem-Clip-Anwendung, zur Reinigung von Gemischen, die Biomoleküle enthalten, zum Testen von Substanzen auf Verunreinigung, zur Erzeugung einer oder als biokatalytische und/oder bioaktive Oberfläche, zu Zellkulturzwecken bzw. der Arbeit mit kultivierten Zellen auf Oberflächen, zur Herstellung eines Implantates. Die Ankopplung von Biomolekülen auf Oberflächen wird derzeit über zwei übergeordnete Methoden realisiert: Bei der ersten Methode werden die Moleküle ungerichtet auf der Oberfläche angebunden (Physisorption). Hierbei werden die Moleküle über elektrostatische Kräfte wie z.B. Wasserstoff-Brückenbindung, van-der-Waals-Kräfte, Dipol-Dipol-Wechselwirkungen oder hydrophobe Interaktionskräfte adsorbiert. Diese Form der Anbindung ist meist nur von geringer Beständigkeit und Bindungsenergie. Zusätzlich erfolgt die Anbindung, da sie ungerichtet ist, häufig so, dass erhebliche Teile der aktiven Gruppen, in der Biomoleküle für die beabsichtigte Reaktion nicht mehr zur Verfügung stehen, da sie aufgrund der Bindungslage sterisch nicht mehr zugänglich sind.

Bei der zweiten Methode erfolgt die Anbindung der Biomoleküle kovalent über entsprechende reaktive Gruppen auf der Oberfläche (Chemisorption). Diese reaktiven Gruppen können z.B. durch Verfahren der Plasmaaktivierung, durch aktive Abscheidungsprozesse oder durch chemische Reaktionen (reaktive Gase oder Flüssigkeiten) erzeugt werden. Es können auch bestimmte Beschichtungen vorgesehen werden, durch die reaktiven Gruppen an der Oberfläche zur Verfügung gestellt werden. In der Literatur ist eine Vielzahl von möglichen reaktiven Gruppen beschrieben. Die wichtigsten Vertreter sind Phosphat-, primäre Amino-, Carboxyl-, Carbamid-, Thiol-, oder quartäre Aminogruppen, wie sie z.B. in der DE 3126551 A1, der US 2004/0209269 A1 oder der WO 9852619 erwähnt sind.

Als Beispiel für eine Einführung aktiver Gruppen auf Oberflächen wird auf die WO 03030129 hingewiesen, bei der eine Silanisierung erfolgt, um reaktive Aminogruppen zu erhalten.

Zur Kopplung komplexer Biomoleküle müssen diese meist durch Vernetzungs-(Cross)-Linker (homo- oder heterofunktionell) mit Oberflächengruppen in Reaktion gebracht werden (vgl. z.B. Hermanson G., Bioconjugate Techniques, Academic Press, London, 1996).

Enthalten die Biomoleküle spezielle Gruppen (auch als Tag bezeichnet), die aufgrund ihrer chemischen Struktur besonders geeignet sind für eine Interaktion mit Teilen einer Oberfläche, so kann der Ort der Anbindung des Biomoleküls aufgrund der Lage des Tags an der Moleküloberfläche gut gesteuert werden. Ein Beispiel hierfür sind die sogenannten His-Tags (Sequenzwiederholung der Aminosäure Histidin), die über komplexierende Interaktionen mit Nickelionen reagieren und das Biomolekül auf der Oberfläche binden. In der Literatur sind aber auch reaktive Oberflächengruppen beschrieben, z. B. Epoxy-, Hydrazin-, Isocyanat-, Catechol- und Azlactongruppen, die direkt z.B. mit den Hydroxyl-, Sulfhydryl-Amino- oder Carboxylgruppen der Biomoleküle reagieren können (vgl. Hermannsson G., Bioconjugate Techniques, Academic Press, London, 1996).

Die Aktivierung der Oberflächen mit diesen Gruppen stellt oft einen chemisch aufwändigen Prozess dar. Darüber hinaus müssen für viele Anwendungen Grundkörper mit einer Oberflächenschicht aufwändig beschichtet oder die Oberflächen anderweitig modifiziert werden, so dass Gegenstände, die für die Kopplung von Biomolekülen eingerichtet sind, häufig in mehrstufigen Verfahren hergestellt werden müssen.

Hinzu kommt, dass viele aus dem Stand der Technik bekannte Aktivierungsverfahren - und dies gilt insbesondere für die verhältnismäßig preisgünstigen - wenig spezifisch sind, so dass häufig nicht nur eine Art von chemischer bzw. physikalischer (aktiver) Bindungsgruppe zur Verfügung gestellt wird. Dies führt aber zu einem oft unerwünschten unspezifischen Bindungsverhalten der anzulagernden Biomoleküle, so dass wiederum ein Teil der Biomoleküle nicht die ideale Ausrichtung erhält, das Biomolekül durch die falsche Anlagerung denaturiert oder sich andere als die erwünschten Biomoleküle anlagern.

Des Weiteren ist problematisch, dass für verschiedene Anwendungen bestimmte Grundmaterialien bevorzugt einzusetzen sind: Diese Grundmaterialien verleihen dem Gegenstand mit der Oberfläche, an die sich Biomoleküle anlagern sollen, z.B. bestimmte mechanische Grundeigenschaften. Wenn man ein verhältnismäßig aufwändiges Beschichtungsverfahren vermeiden möchte, bedeutet dies, dass für jedes Grundmaterial eine eigene Bindungschemie zu jedem (gewünschten) Biomolekül oder wenigstens jeder (gewünschten) Gruppe von Biomolekülen entwickelt werden muss.

Dies gilt insbesondere auch dann, wenn an der Zusammensetzung des Grundmaterials Änderungen vorgenommen werden, die bereits lediglich die Änderung der Einfärbung des Materials betreffen können.

Hinzu kommt, dass für eine Reihe von Materialien - dies gilt auch für eine Reihe von Kunststoffen - eine geeignete Bindungschemie zu einer Vielzahl von Biomolekülen noch nicht entwickelt wurde.

Aufgabe der vorliegenden Erfindung war es vor dem Hintergrund des Standes der Technik daher, ein System anzugeben, das ohne großen Aufwand an erwünschte Grundanforderungen, wie z.B. mechanische Stabilität oder Wärmeleitfähigkeit angepasst werden kann, das gleichzeitig aber ebenfalls ohne großen Aufwand an die Bindungserfordernisse für die Bindung von gewünschten Biomolekülen adaptiert ist. Bevorzugt sollten diesem System entsprechende Gegenstände preisgünstig und/oder in möglichst wenigen Arbeitsschritten herstellbar sein.

Diese Aufgabe wird gelöst durch einen Formkörper, umfassend eine Matrix aus einem Material, ausgewählt aus der Gruppe bestehend aus Metall, Keramik und polymerem Kunststoff, und in die Matrix eingebettete Kopplungspartikel, wobei ein Teil der Oberfläche des Formkörpers in einer geometrischen Form oder einem regelmäßigen Muster und/oder eine Fläche des Formkörpers vollständig oder die gesamte Oberfläche des Formkörpers mechanisch bearbeitet ist.

"Matrix" im Sinne dieses Textes ist das Material, das im Wesentlichen formgebend für den Formkörper ist. Im Regelfall und bevorzugt besteht der Formkörper überwiegend aus dem Matrixmaterial, bevorzugt zu ≥ 80%, weiter bevorzugt ≥ 90%. Das Matrixmaterial kann grundsätzlich auch ein Mischmaterial aus verschiedenen Werkstoffen sein.

"Kopplungspartikel" im Sinne dieses Textes sind Partikel, die sich in der stofflichen Zusammensetzung vom Matrixmaterial unterscheiden. Der Fachmann wird die Partikelform und die Partikelgröße dem jeweiligen Zweck anpassen. Bevorzugt liegt die Größe des größten Durchmessers der Kopplungspartikel im Bereich von 10 nm bis 500 µm, weiter bevorzugt 50 nm bis 100 µm, besonders bevorzugt 100 nm bis 5 µm, jeweils bezogen auf das arithmetische Mittel der Größenverteilung.

Die Partikel können hierbei auch in anisotropischen Formen (Stäbchen, Nadeln, Ovale, etc.) vorliegen. Bestimmung der Partikelgröße erfolgt mit mikroskopischen Techniken. Der Fachmann wird hierbei die jeweils geeigneten Methoden anwenden, um alle Partikelgrößen und deren Größenverteilung bestimmen zu können. Bevorzugt erfolgt die Bestimmung des vorgenannten arithmetischen Mittels der Größenverteilung wie folgt: In die Berechnung gehen ausschließlich die Partikel ein, die im Lichtmikroskop einen größten Durchmesser von ≥ 10 µm aufweisen, sowie die Partikel, die im Rasterelektronenmikroskop einen größten Durchmesser von < 10 µm und ≥ 100 nm aufweisen und diejenigen Partikel, die im Transmissionselektronenmikroskop einen größten Durchmesser < 100 nm aufweisen.

Dem Fachmann ist klar, dass bei der Mikroskopie von nicht-kugelförmigen Partikeln die Orientierung des einzelnen Partikels für das Ergebnis des jeweiligen größten (mikroskopisch gemessenen) Durchmessers eine Rolle spielen kann. Für das Auswerten des arithmetischen Mittels werden bevorzugt jedoch lediglich die in der jeweiligen Messung tatsächlich gemessenen größten Partikeldurchmesser berücksichtigt. Dies gilt selbstverständlich dann nicht, wenn bei nicht-sphärischen Partikeln eine gezielte Ausrichtung der Partikel stattgefunden hat. In diesem Fall muss der Schnitt für die mikroskopische Messung so erfolgen, dass der tatsächlich größte Durchmesser der (ausgerichteten) Partikel durch die jeweilige Mikroskopiemethode erfasst werden kann.

Ferner ist es bevorzugt, dass sich Partikelmaterial und Matrixmaterial deutlich voneinander unterscheiden, zum Beispiel indem Partikelmaterial und Matrixmaterial jeweils aus einer anderen der nachfolgenden Materialgruppen gewählt werden: Metalle/ Metalllegierungen, Metalloxide sowie deren Legierungen, Keramiken, Polymere, insbesondere Kunststoffe, anorganische Gläser.

Ein "Formkörper" im Sinne dieses Textes ist ein geometrischer Körper, der durch ein formgebendes Fertigungsverfahren wie insbesondere Spritzguss, Prägen, Spitzprägen, Pressen und Sintern, Schlickerguss hergestellt wird.

"Metall" im Rahmen dieser Erfindung bedeutet Reinmetalle und Legierungen.

Abhängig vom Herstellungsverfahren des Formkörpers müssen die Kopplungspartikel ausgestaltet sein. Dabei ist es bevorzugt, dass die Kopplungspartikel einen höheren Schmelzpunkt als das Matrixmaterial besitzen, insbesondere für Verfahren, bei denen die Formkörper unter Schmelzen oder Sintern des Matrixmaterials hergestellt werden (vgl. auch weiter unten).

Sofern das Matrixmaterial im Wesentlichen keramisches Material ist, muss der Fachmann selbstverständlich die Kopplungspartikel so wählen, dass diese kein Bestandteil der

Keramik während des Herstellungsprozesses des Formkörpers werden. Dies schließt grundsätzlich nicht aus, dass z.B. in eine keramische Matrix ebenfalls keramische Kopplungspartikel eingebettet werden. Es ist hierbei lediglich zu beachten, dass Kopplungspartikel und Matrix räumlich voneinander unterscheidbar sind.

Bevorzugte Herstellungsverfahren für Formkörper mit einer Matrix aus Metall sind Pulvermetallurgische Verfahren wie Pressen oder Sintern, einschließlich, aber nicht beschränkt auf Metallpulverspritzguß, Pastendruck, Prägen oder Drucken.

Bevorzugte Herstellungsverfahren für Formkörper mit einer Matrix aus Keramik sind Pressen, Sintern, Keramik-Spritzguß, Schlickerguß oder -druck sowie Freeze Casting.

Unter bestimmten Umständen kann es auch bevorzugt sein, dass der erfindungsgemäße Formkörper ein Mehrkomponentenkörper ist, also aus unterschiedlichen Matrixmaterialbereichen wie zum Beispiel Kunststoff, Keramik und/oder Metall besteht. Besonders bevorzugt in diesem Zusammenhang ist, dass nur eins der erwähnten Matrixmaterialien bei einem erfindungsgemäßen Mehrkomponentenformkörper mit Kopplungspartikeln versetzt ist.

Unter dem Merkmal, dass "der Formkörper mechanisch bearbeitet ist", ist im Sinne dieser Erfindung zu verstehen, dass der Formkörper nach seinem eigentlichen Herstellungsprozess einer mechanischen Nachbearbeitung unterworfen worden ist, wobei Material von dem Formkörper abgetragen wird. Bevorzugte Abtragungsverfahren im Sinne dieser Erfindung sind Schleifen, Fräsen und/oder Strahlen, Polieren, Laserabtrag, (CO₂-) Schnee-Strahlen. Dabei hinterlassen die Bearbeitungsverfahren auf dem Formkörper Spuren, durch die der Fachmann das eingesetzte Verfahren erkennen kann.

Unter dem Merkmal, dass "ein Teil der Oberfläche des Formkörpers in einer geometrischen Form oder einem regelmäßigen Muster mechanisch bearbeitet ist", ist zu verstehen, dass eine gezielte Oberflächenbehandlung vorgenommen worden ist, die sich von zufälligen Schleifspuren, wie sie z.B. im Gebrauch entstehen können, unterscheidet. Bevorzugt sind unter geometrischen Formen in diesem Zusammenhang die klassischen geometrischen Formen, wie Kreise, Quadrate, Rechtecke, Trapeze, Parallelogramme, Oktaeder, Tetraeder, Polyeder zu verstehen. Dabei können diese geometrischen Formen selbstverständlich auch dreidimensional ausgestaltet sein, z.B. als Kugelabschnitt

("Well"). Unter einem regelmäßigen Muster ist im Sinne der Anmeldung zu verstehen, dass die Bearbeitungsbereiche repetitive Formen umfassen.

Auch das Merkmal, dass die "Gesamtoberfläche oder eine Fläche des Formkörpers vollständig mechanisch bearbeitet ist", unterscheidet die erfindungsgemäßen Formkörper von Formkörpern, die lediglich Gebrauchsspuren aufweisen.

Die oben gestellte Aufgabe wird bevorzugt gelöst durch einen Formkörper, umfassend eine polymere Kunststoffmatrix und darin eingebettete Kopplungspartikel, wobei der Formkörper mechanisch bearbeitet ist, so dass ein Teil der Partikel wenigstens teilweise von der Kunststoffmatrix unbedeckt ist, und wobei ein Teil der Oberfläche des Formkörpers in einer geometrischen Form oder einem regelmäßigen Muster und/oder eine Fläche des Formkörpers vollständig oder die gesamte Oberfläche des Formkörpers mechanisch bearbeitet ist.

Eine polymere Kunststoffmatrix im Sinne dieses Textes ist eine Matrix aus klassischen Polymeren mit repetitiven gleichen oder unterschiedlichen Untereinheiten.

Auch das Merkmal, dass die "Gesamtoberfläche oder eine Fläche des Formkörpers vollständig mechanisch bearbeitet ist", unterscheidet die erfindungsgemäßen Formkörper von Formkörpern, die lediglich Gebrauchsspuren aufweisen.

Der Vorteil der erfindungsgemäßen Formkörper liegt insbesondere darin, dass Materialien mit zwei Grundfunktionen miteinander kombiniert werden: Zum einen handelt es sich hierbei um die Matrix (bevorzugt die polymere Kunststoffmatrix), die für die mechanischen Grundeigenschaften sowie wie für weitere Eigenschaften, wie z.B. eine Einstellung von elektrischer- und/oder thermischer Leitfähigkeit und/oder magnetische Eigenschaften, sorgt. Zum anderen handelt es sich um die Kopplungspartikel, die hinsichtlich ihrer Oberfläche auf das gewünschte Bindungssystem eingerichtet sind. Damit ist es möglich, für die Anbindung eines bestimmten Ziel(bio)moleküls stets die gleiche Art von Kopplungspartikeln zu verwenden, während die Matrix wiederum an die weiteren Umstände des geplanten Einsatzes des erfindungsgemäßen Formkörpers angepasst werden kann. Dabei können diese Komponenten über weite Strecken beliebig miteinander kombiniert werden.

Im Stand der Technik wurde diese unterschiedliche Problematik oft durch Beschichtung eines Grundkörpers gelöst, was - wie bereits oben angedeutet - einen zusätzlichen Arbeitsschritt voraussetzt. Letztendlich ist durch den erfindungsgemäßen Formkörper die Kopplungschemie weitestgehend unabhängig vom Matrix- und Basis-Material, obwohl faktisch nur eine Materialphase benötigt wird, d.h. der Formkörper im Wesentlichen homogen ausgestaltet sein kann (eine Gleichverteilung der Kopplungspartikel liegt vor).

Vorteilhaft ist auch, dass die bevorzugten erfindungsgemäßen Formkörper mit gängigen Kunststoffherstellungsverfahren und insbesondere Formungsverfahren hergestellt werden können. Ein erfindungsgemäß zu lösendes Problem war in diesem Zusammenhang, dass bei den üblichen Herstellungsverfahren für Formkörper die Kunststoffmatrix verflüssigt wird. Da im Rahmen des Herstellungsprozesses die Kopplungspartikel in der flüssigen Matrix dispergiert werden, führt dies regelmäßig zu einem vollständigen Einschluss der Partikel in die Kunststoffmatrix, da aufgrund von Kompoundierungs- und Oberflächeneffekten der flüssige Kunststoff die Partikel stets vollständig umschließt. Ähnlich verhalten sich hierzu oft Metall- und Keramikmatrices, wobei hier nach der Formgebung des Formkörpers noch ein Sinterschritt nach dem Metallpulver - oder Keramikspritzguß erfolg, um den Zusammenhalt des Formkörpers zu erhöhen. Eingebette Kopplungspartikel wie Metallpartikel in Keramikmatrices oder Keramikpartikel in Metallmatrices können ebenso zu verbesserten Materialeigenschaften führen.

Insbesondere, wenn es auf eine homogene Verteilung der Partikel ankommt, dauert die Dispergierung der Partikel in der flüssigen Kunststoffmatrix regelmäßig mehrere Minuten. Dabei entsteht eine dünne Polymerhaut auf den Partikeln, die auf die Partikel im Wesentlichen vollständig umschließt, die beim Herstellprozess des Formkörpers letztendlich an der Oberfläche liegen.

Durch diesen Einschluss würden die Kopplungspartikel einer Anbindung nicht zur Verfügung stehen, da die Oberfläche des Formkörpers ausschließlich von Matrixmaterial gebildet wird. Gelöst wird dieses Problem erfindungsgemäß durch gezieltes mechanisches Bearbeiten des Formkörpers. Dabei kann es vorteilhaft sein, nur bestimmte Bereiche des Formkörpers zu bearbeiten, so dass Regionen mit einer hohen Konzentration von freiliegenden Kopplungspartikeln vorliegen, während andere Bereiche, die nicht bearbeitet wurden, praktisch keine Kopplungspartikel an der Oberfläche zur Verfügung stellen. Damit lässt sich der Ort der Bindung der gewünschten Biomoleküle auf dem Formkörper optimal steuern. Bevorzugt ist in diesem Zusammenhang, dass das Matrixmaterial gegenüber den anzubindenden Biomolekülen weitgehend und weiter bevorzugt vollständig inert ist.

Das mechanische Bearbeiten bietet aber noch weitere Vorteile, auch für den Fall, dass das Matrixmaterial kein Kunststoffmaterial umfasst: So führt ein geeignet ausgeführtes mechanisches Bearbeiten dazu, dass die Kopplungspartikel weniger stark abgetragen werden als das Matrixmaterial. Dies führt dazu, dass die für eine Kopplung zur Verfügung stehende Fläche relativ zur Oberfläche des Formkörpers vergrößert wird. Des Weiteren ist es durch das mechanische Bearbeiten möglich, Bereiche bevorzugter Bindung auf der Oberfläche des Formkörpers zu erzeugen. Dies geschieht zum einen dadurch, dass im Bereich der mechanischen Bearbeitung eine relativ erhöhte Bindungsfläche zur Verfügung gestellt wird, zum anderen kann das mechanische Bearbeiten so ausgeführt werden, dass dreidimensionale Strukturen (wie z.B. der Wells) ausgebildet werden, in die Flüssigkeiten mit zu bindenden Molekülen (zu koppelnden Molekülen) eingebracht werden können, so dass die faktisch auftretende Bindung auf den Bereich der Vertiefung konzentriert ist und nicht (z.B. ungewünschte) Bereiche des Formkörpers betrifft.

Wie bereits oben angedeutet, ist es aufgrund des erfindungsgemäßen Formkörpers möglich, eine Vielzahl von Materialien als Matrixmaterial einzusetzen, die aufgrund ihrer Chemie bislang nicht für Bindungsanwendungen für Biomoleküle geeignet waren, zumindest nicht für Bindungsanwendungen ohne eine zusätzliche Beschichtung.

Ein weiterer Vorteil der Erfindung ist darin zu sehen, dass über den Füllgrad und die Partikelgröße gezielt die Kopplungsdichte von Biomolekülen auf den erfindungsgemäßen Formkörper eingestellt werden kann. Darüber hinaus ist es möglich, durch den Einsatz unterschiedlicher Kopplungspartikel, also Kopplungspartikel, die über eine zueinander verschiedene Kopplungschemie verfügen, gezielt mehr als eine Spezies (oder eine Art von Spezies) von Biomolekülen zu binden. Da auch die Konzentration der unterschiedlichen Kopplungspartikel jeweils separat eingestellt werden kann, ist es somit auch möglich, die Konzentration der Beladung der Oberfläche des erfindungsgemäßen Formkörpers mit bestimmten Biomolekülen unabhängig voneinander zu steuern, sofern die Biomoleküle über eine unterschiedliche Bindungschemie verfügen.

Des Weiteren ist es insbesondere für Matrixmaterial aus polymeren Kunststoffen möglich, das Verhältnis von spezifischen zu unspezifischen Bindungen wesentlich zu verbessern: Selbst wenn für das Matrixmaterial ein Kunststoff gewählt wird, der nicht vollständig inert gegenüber den zu bindenden Biomolekülen (oder anderen Biomolekülen, denen der Formkörper ausgesetzt wird) ist, so kann durch die gezielt ausgewählte Bindungschemie der Kopplungspartikel eine besonders hohe Affinität zu den erwünschten Biomolekülen eingestellt werden, so dass in den Bereichen, in denen die Kopplungspartikel durch die mechanische Bearbeitung freigelegt wurden, eine wesentliche Anreicherung der Biomoleküle erfolgt.

Für die Herstellung des Formkörpers wird der Fachmann das gewünschte Herstellverfahren, das Matrixmaterial und die Kopplungspartikel, hier insbesondere deren Größe und/oder Ausformung aneinander anpassen. Hierzu bestehen z.B. im Bereich des Spritzgusses umfangreiche Erfahrungen.

Ein Vorteil des bevorzugt erfindungsgemäßen Formkörpers liegt auch darin, dass durch den Einsatz der oft günstigen Kunststoffe teurere Materialien ersetzt werden können: So können z.B. anstelle von Vollglasoberflächen Kunststoffmaterialien als Matrixmaterialien eingesetzt und als Kopplungspartikel Glaspartikel verwendet werden. Hinzu kommt noch, dass mit Kunststoffformkörpern Formen erzeugt werden können, die z.B. mit Glas nur unter hohem Aufwand oder gar nicht erzeugbar sind.

Durch die Tatsache, dass im bevorzugten erfindungsgemäßen Formkörper das Matrixmaterial und die Kopplungspartikel nebeneinander vorliegen, ist es möglich, sämtliche Vorteile von Kunststoffen für verschiedene Einsatzzwecke auszunutzen. Gleichzeitig ist es möglich, die weit entwickelten, und sehr häufig sehr preisgünstigen Kunststoffherstellungsverfahren bzw. Kunststoffformungsverfahren einzusetzen, um die erfindungsgemäßen Formkörper zu erstellen. Ein vergleichbarer Ansatz ist aus dem Stand der Technik nicht bekannt. Zwar beschreibt die US 5,993,935 A eine poröse Matrix mit Partikeln zur Molekülanbindung. Bei diesen Matrizen handelt es sich jedoch um mikroporöse Membransysteme, die keine Formkörper im Sinne dieser Erfindung sind. Auch die in dem genannten Dokument beschriebenen gewebten oder ungewebten Gewebe sind nicht als Formkörper im Sinne der vorliegenden Erfindung zu verstehen. Außerdem gibt es in diesem Dokument keinen Hinweis auf eine mechanische Bearbeitung des Matrixmaterials zur Freilegung von Kopplungspartikeln.

Bevorzugte erfindungsgemäße Formkörper sind undurchlässig für Wasser, was gewährleistet, dass die gewünschte Anbindung der Biomoleküle ausschließlich an der

Oberfläche (und hier bevorzugt im Bereich, wo der Formkörper mechanisch bearbeitet worden ist) erfolgt.

Bevorzugte erfindungsgemäße Formkörper haben eine Mindestdicke von 5 µm, bevorzugt ≥ 100 µm, weiter bevorzugt ≥ 1cm und besonders bevorzugt ≥ 10cm.

Bevorzugt ist ein erfindungsgemäßer Formkörper, bei dem wenigstens 1%, bevorzugt ≥ 5%, weiter bevorzugt ≥ 10% und besonders bevorzugt ≥ 25% einer Fläche des Formkörpers mechanisch bearbeitet sind. Gleichzeitig kann bevorzugt sein, dass maximal 80%, bevorzugt maximal 70%, weiter bevorzugt maximal 50% der Oberfläche des Formkörpers bearbeitet sind. Je mehr Fläche mechanisch bearbeitet ist, desto mehr Kopplungspartikel werden an der Oberfläche des erfindungsgemäßen Formkörpers freigelegt. Dementsprechend lässt sich die erzielbare Ladung des Formkörpers mit Biomolekülen auch durch die Fläche steuern, die mechanisch bearbeitet worden ist.

Es sei in diesem Zusammenhang noch darauf hingewiesen, dass die eingesetzten Kopplungspartikel häufig teurer als das Matrixmaterial sind. Dementsprechend ist es häufig erwünscht, dass der Formkörper über ein großes Oberflächen-Volumenverhältnis verfügt, so dass ein möglichst hoher Anteil an Kopplungspartikeln für eine Bindung verfügbar gemacht werden kann. Auf der anderen Seite ermöglicht eine Verteilung der Kopplungspartikel über den gesamten Formkörper grundsätzlich wesentliche Vereinfachungen im Herstellprozess, so dass höhere Kosten für Materialeinsatz an Kopplungspartikeln durch die Vorteile bei der Herstellung der Formkörper ausgeglichen werden.

Darüber hinaus besitzt eine homogene Verteilung der Kopplungspartikel den Vorteil, dass die mechanische Bearbeitung auch dreidimensional in den Formkörper (also in die Tiefe) hineingeführt werden kann, so dass z.B. Vertiefungen, wie Wells (siehe oben) ausgeprägt werden können. Auf diese Art ist gewährleistet, dass trotz der dreidimensionalen Bearbeitung immer eine gleichmäßige Konzentration an Bindungsstellen pro Oberflächeneinheit zur Verfügung steht. Dies ist beispielsweise bei komplizierten dreidimensionalen Oberflächengestaltungen nicht oder nur schwer gewährleistbar, wenn die Bindungsstellen für die Bindungsmoleküle erst durch eine zusätzliche Beschichtung auf einen Körper aufgebracht werden: Es ist nicht immer einfach, eine vollständige Benetzung in dreidimensionalen Strukturen mit einer neuen Schicht zu gewährleisten. Bevorzugt ist ein erfindungsgemäßer Formkörper, der so mechanisch bearbeitet ist, dass ein Teil der Partikel aus der Oberflächenebene des Formkörpers herausragt. Dies ist möglich, in dem bei der mechanischen Bearbeitung das Matrizenmaterial so abgetragen wird, dass die Kopplungspartikel nicht oder nur zu einem verringerten Maße betroffen sind. Dies führt dazu, dass eine höhere Oberfläche des Kopplungspartikels zur Bindung an die Biomoleküle zur Verfügung steht. Selbstverständlich ist dabei zu beachten, dass für die Kopplungspartikel noch eine ausreichende Einbettung in das Matrixmaterial besteht.

Bei bevorzugten erfindungsgemäßen Formkörpern umfasst die Kunststoffmatrix Material, ausgewählt aus Kunststoffen bestehend aus Thermoplasten, Duroplasten und Elastomeren. Für einige Anwendungen ist es bevorzugt, dass die Kunststoffmatrix aus diesem Material besteht.

Bevorzugt ist das Matrixmaterial ausgewählt aus der Gruppe bestehend aus Polypropylen (PP), Polystyrol (PS), Polyurethan (PU), Polycarbonat (PC), Polymethylmethacrylat (PMMA), Polyoxymethylen (POM), Polyvenylchlorid (PVC), Polyethylen (PE), Thermoplastischer Polyurethan (TPU), Polyetheretherketon (PEEK), Polytetrafluorethylen (PTFE) und Biopolymeren, insbesondere thermoplastische Stärke und PLA (Polymilchsäure).

Erfindungsgemäß bevorzugt ist ein Formkörper, wobei die Kopplungspartikel ein Material umfassen oder aus einem Material bestehen, ausgewählt aus der Gruppe bestehend aus Metall, Metalloxid, Keramik, Glas und Kunststoffen mit höherer Schmelztemperatur als das Matrixmaterial.

Bevorzugte Materialien für Metalle (als Kopplungspartikel) sind in diesem Zusammenhang Gold (Au), Cobalt (Co), Zink (Cn), Wolfram, Eisen (Fe), Kupfer (Cu), Nickel (Ni), Nickel, Magnesium (Mg), Aluminium (Al), Titan (Ti) oder deren Legierungen wie z.B., Stahl oder Edelstahl. Die genannten Metalle kommen auch als Matrix-Material in Frage.

Bevorzugte Materialien für Keramiken (als Kopplungspartikel) sind Keramiken auf Basis von Kalziumcarbonat, Aluminiumoxid, Hydroxylapatit, Zirkondioxid, Titandioxid, Indium Zinn Oxid (ITO), Bariumoxid oder Calziumphosphate.

Bevorzugte Gläser (als Kopplungspartikel) sind siliziumdioxidbasiertes Glas und Silicatglas, die im weiteren Verlauf auch als Glaskugel bzw. Glaspartikel bezeichnet werden.

Bevorzugte Polymere (als Kopplungspartikel) sind PC, PEEK, PMMA bzw. können aus der Liste der Matrixmaterialien ausgewählt werden, insofern die Kombination einen höheren Schmelzpunkt der Kopplungspartikel als die Matrix berücksichtigt. Es sind eine Vielzahl von Verfahren zur Oberflächenmodifizierung bekannt, die die Verbesserung der Kopplungseigenschaften an (bestimmte) Biomoleküle zu bewirken. Dies gilt insbesondere auch für die vorbeschriebenen bevorzugten Materialen, die in Kopplungspartikeln enthalten sein können. Dementsprechend umfasst ein bevorzugter erfindungsgemäßer Formkörper Kopplungspartikel, die zumindest im Bereich, wo sie von der Matrix unbedeckt sind, wenigstens teilweise zur Verbesserung der Kopplungseigenschaften an Biomoleküle oberflächenmodifiziert sind.

Unter Kopplungspartikel werden nicht nur Partikel mit von kugelartigem, plättchenhaften, zylindrischem bzw. röhrenartigem, sondern auch solche mit faserartigem Aussehen bzw. Struktur verstanden.

Bevorzugt werden im Rahmen der Oberflächenmodifizierung der Kopplungspartikel funktionelle Gruppen eingeführt. Bevorzugte funktionelle Gruppen sind z. B. Epoxy-, Hydrazin-, Isocyanat-, Catechol- und Azlactongruppen, sowie Hydroxyl-, Sulfhydryl-, Amino- uder Carboxylgruppen auf der Kopplungspartikeloberfläche.

Dabei kann die Oberflächenmodifizierung vor Herstellung des Formkörpers erfolgen. Dies bedeutet, dass in das Herstellungsverfahren für den erfindungsgemäßen Formkörper bereits oberflächenmodifizierte Kopplungspartikel eingebracht werden. Es kann aber auch bevorzugt sein, eine entsprechende Oberflächenmodifizierung erst nach Herstellen des Formkörpers durchzuführen, z.B. wenn die zur Modifizierung zu verwendenden Chemikalien teuer sind. Dann brauchen diese nur in geringerer Konzentration eingesetzt werden, da sie lediglich auf die nach der mechanischen Bearbeitung freiliegenden Flächen der Kopplungspartikel gebunden werden müssen.

Bevorzugte Verfahren zur Oberflächenmodifizierung der Kopplungspartikel sind Silanisierung, insbesondere mit Amino-, Epoxy- oder Carboxylsilanen (u.a. modifizierten Silanen) Aufbringen spezifischer Schichten, z.B. Dihydroxyphenylalanin (DOPA), die Anbindung der Biomoleküle über Carbonsäuren, Amino-, Epoxy-, Hydroxyl-, Isocyanat-, Zucker-, Photoreaktiv- oder Sulfhydrylgruppen auf den Kopplungspartikeln ermöglichen. Dabei wird der Fachmann selbstverständlich den geeigneten Zeitpunkt für das Aufbringen der Bindungschemie (Oberflächenmodifizierung) wählen: Sofern für die gezielte Anbindung der gewünschten Biomoleküle empfindliche Gruppen benötigt werden, bietet es sich an, die Oberflächenmodifizierung erst nach Herstellen des erfindungsgemäßen Formkörpers vorzunehmen.

Im Sinne der Erfindung ist es bevorzugt, dass die Oberflächenmodifizierung der Kopplungspartikel (zur Einführung funktionaler Gruppen) insbesondere nach Herstellen der Formkörper so erfolgt, dass die Modifizierung verglichen mit dem Matrixmaterial die Kopplungspartikel deutlich stärker, bevorzugt ausschließlich betrifft. Deutlich stärker heißt in diesem Zusammenhang folgendes: Beim Einbringen von funktionalen Gruppen/Linkern im Rahmen der Oberflächenmodifzierung der Kopplungspartikel sind das Kopplungspartikelmaterial, das Matrixmaterial, die an die Kopplungspartikel anzubindenden funktionalen Gruppen und/oder die Reaktionsbedingungen so gewählt, dass an die Kopplungspartikel ≥ 50 %, bevorzugt ≥ 75%, weiter bevorzugt ≥ 99% der funktionelle Gruppen pro Flächeneinheit angebunden werden.

Erfindungsgemäß bevorzugt ist ein erfindungsgemäßer Formkörper herstellbar oder hergestellt unter Einsatz eines oder mehrerer Verfahren wie Spritzgussverfahren oder Spritzprägen, durch Extrusion, Heißprägen, Prägen oder Formgießen und nachfolgendes mechanisches Bearbeiten.

Besonders bevorzugt ist in diesem Zusammenhang der Einsatz eines Spritzgussverfahrens. Die Tatsache, dass der erfindungsgemäße Formkörper durch diese gängigen und günstigen Verfahren hergestellt werden kann, ist insbesondere aus dem Kostengesichtspunkt heraus ein wesentlicher Vorteil der Erfindung. Zusätzlich sind aber durch diese typischen Verfahren besonders einfach dreidimensionale Ausgestaltungen möglich und die Verfahren sind etabliert auch für die Massenherstellung, so dass auch große Mengen der erfindungsgemäßen Formkörper erfindungsgemäß erzeugt werden können.

Besonders bevorzugte Verfahren für die Herstellung erfindungsgemäßer Formkörper sind Verfahren, in denen mehr als eine Komponente eingesetzt werden können. Hier ist bevorzugt zu nennen der Zwei- oder Mehrkomponentenspritzguß. Die in dem

Mehrkomponentenverfahren zur Herstellung des erfindungsgemäßen Formkörpers eingesetzten Komponenten können dabei
a) eine Komponente sein, die Kopplungspartikel umfasst und eine weitere Komponente, die keine Kopplungspartikel umfasst,
b) zwei Komponenten sein, die jeweils einen anderen Kopplungspartikel umfassen,
c) Komponenten sein, die zueinander verschiedene Kunststoffmatrixmaterialien umfassen und/oder
d) Komponenten, die sich hinsichtlich ihrer Matrixmaterialzusammensetzung und hinsichtlich der darin enthaltenen Kopplungspartikel unterscheiden.

Der Vorteil dieser Mehrkomponentenverfahren ist darin zu sehen, dass einerseits der Einsatz an teuren Materialien (im Regelfall werden dies die Kopplungspartikel sein) reduziert werden kann, andererseits je nach Bedarf verschiedene Zonen innerhalb des erfindungsgemäßen Formkörpers erzeugt werden können, die unterschiedliche Eigenschaften und unterschiedliche Funktionalisierungen besitzen. Dabei können die Kombinationen der Kopplungspartikel mit dem Matrixmaterial innerhalb eines Formkörpers auf verschiedene Funktionen der Teilbereiche eines Formkörpers abgestimmt werden. So ist es beispielsweise häufig nicht erforderlich, dass sich Kopplungspartikel im Inneren des Formkörpers befinden, da sie ihre Funktion an der Oberfläche ausüben. Damit kann bei einer entsprechenden Ausgestaltung des Formgebens im Herstellungsverfahren für den erfindungsgemäßen Formkörper der Materialeinsatz an den im Regelfall teureren Kopplungspartikeln verringert werden, indem z.B. ein "Kern" des Formkörpers aus einer Komponente erzeugt wird, die Kopplungspartikel frei ist. Außerdem können verschiedene Zonen auf dem erfindungsgemäßen Formkörper erzeugt werden, die je nach Materialkombination von Kopplungspartikel und Matrixmaterial unterschiedliche Funktionen ausüben, wie z.B. die Bindung bestimmter Moleküle.

Bestandteil der Erfindung ist ein erfindungsgemäßer Formkörper, umfassend an seine Oberfläche gekoppelte Biomoleküle. Bevorzugt sind diese Biomoleküle ausgewählt aus der Gruppe bestehend aus Proteinen, Peptiden, Kohlenhydraten, Lipiden, Nukleinsäuren, Hormonen, Aminosäuren und Nukleotiden.

Biomoleküle im Sinne der vorliegenden Erfindung sind Moleküle, die in Organismen hergestellt werden können.

Ein entsprechender bevorzugter erfindungsgemäßer Formkörper, an den Biomoleküle gekoppelt sind, kann eine Vielzahl von Funktionen erfüllen. Er kann als Biosensor eingesetzt werden, kann für chemische, biochemische oder immunologische Nachweisreaktionen verwendet werden, er kann im Bereich der qualitativen und quantitativen Analyse eingesetzt werden, als Sensor dienen, als Adhäsionsmedium für Zellkulturen oder als Teil eines Implantates. Grundsätzlich ermöglicht ein solcher bevorzugter erfindungsgemäßer Formkörper eine Vielzahl von Anwendungen, die Biomoleküle an der Oberfläche erfordern.

Bevorzugt ist ein erfindungsgemäßer Formkörper, der an seiner Oberfläche angekoppelte Biomoleküle umfasst, wobei die Biomoleküle zu ≥ 70% bevorzugt ≥ 85%, weiter bevorzugt ≥ 95% besonders bevorzugt ≥ 99% an die Kopplungspartikel gekoppelt sind, bezogen auf die Menge aller an den Formkörper gekoppelten Biomoleküle.

Ein solcher bevorzugter erfindungsgemäßer Formkörper ist so ausgestaltet, dass ein Großteil der Biomoleküle, denen er ausgesetzt ist, sich an die Kopplungspartikel anlagert. Dies kann - wie bereits angedeutet - durch eine entsprechende Auswahl des Matrixmaterials gewährleistet werden. Auf diese Art werden unspezifische und/oder unerwünschte Bindungen an den Formkörper vermieden.

Wie bereits weiter oben angedeutet kann der Formkörper so ausgestaltet werden, dass eine Vielzahl von definierten Bindungszonen besteht. So kann die mechanische Bearbeitung der Oberflächen so erfolgen, dass die Bindungszone in Form von Wells entsteht, die miteinander nicht in Kontakt stehen. Auf diese Art ist es möglich, getrennte Reaktionsräume zu schaffen, die jeder mit jeweils gleichen oder zueinander verschiedenen Biomolekülen beladen werden können. Diese können wiederum jede für sich einer Probe ausgesetzt werden, die für jeden Reaktionsraum identisch zusammengesetzt ist oder sich von Reaktionsraum zu Reaktionsraum unterscheiden kann. Damit sind Massentests - auch automatisiert - hervorragend möglich. Eine weitere Flexibilisierung wird in dieses System dadurch gebracht, dass in den einzelnen Reaktionsräumen auf die Kopplungspartikel Bindungsgruppen aufgebracht werden, die sich von denen auf den Partikeln in anderen Reaktionsräumen unterscheiden.

Bevorzugt ist ein erfindungsgemäßer Formkörper, an den die Biomoleküle über Komplexierung oder kovalent angekoppelt sind. In diesem Zusammenhang ist selbstverständlich bevorzugt, dass ein Großteil der Biomoleküle an die Kopplungspartikel gekoppelt sind. Diese bevorzugte Form der Kopplung führt zu einer dauerhaften Bindung, die für viele Anwendungen erforderlich ist. Des Weiteren ist es im Prinzip möglich, die Zahl der Bindungsstellen durch geeignete Verfahren z.B. durch Erzeugen einer bestimmten Beladungsdichte mit reaktiven Gruppen auf den Kopplungspartikeln zu kontrollieren sowie eine zielgerichtete Ankopplung zu ermöglichen.

Teil der Erfindung ist auch ein Verfahren zum Herstellen eines erfindungsgemäßen Formkörpers, umfassend die Schritte:
a) Bereitstellen eines Kunststoffmaterials und/oder eines Kunststoffvorläufermaterials und/oder eines Metallmaterials und/oder eines keramischen Vorläufermaterials,
b) Bereitstellen von Kopplungspartikeln,
c) Mischen der Kopplungspartikel mit dem Kunststoffmaterial und/oder dem Kunststoffvorläufermaterial und/oder dem Metallmaterial und/oder dem keramischen Vorläufermaterial,
d) Formen eines Formkörpers aus der Mischung
e) ggf. im Fall von Metallen und Keramiken das Ausbrennen von ggf. eingesetzten polymeren oder wachsartigen Bindermaterials (Entbindern).
f) ggf. im Fall von und Metallen und einigen Keramiken das Sintern zum Härten und Verdichten des Matrixmaterials bei hohen Temperaturen
g) und mechanisches Bearbeiten des Formkörpers, so dass ein Teil der Oberfläche des Formkörpers in einer geometrischen Form oder einem regelmäßigen Muster und/oder eine Fläche des Formkörpers vollständig oder die gesamte Oberfläche des Formkörpers mechanisch bearbeitet ist.

Ein bevorzugtes erfindungsgemäßes Verfahren zum Herstellen eines erfindungsgemäßen polymeren Formkörpers umfasst dabei die folgenden Schritte:
a) Bereitstellen eines Kunststoffmaterials und/oder eines Kunststoffvorläufermaterials (z.B. Monomere oder Oligomere) für eine Kunststoffmatrix
b) Bereitstellen von Kopplungspartikeln,
c) Mischen der Kopplungspartikel mit dem Kunststoffmaterial und/oder dem Kunststoffvorläufermaterial (z.B. Monomere oder Oligomere),
d) Formen eines Formkörpers aus der Mischung und
e) mechanisches Bearbeiten des Formkörpers, so dass ein Teil der Partikel wenigstens teilweise von der Kunststoffmatrix unbedeckt ist, und ein Teil der Oberfläche des Formkörpers in einer geometrischen Form oder einem regelmäßigen Muster und/oder eine Fläche des Formkörpers vollständig oder die gesamte Oberfläche des Formkörpers mechanisch bearbeitet ist.

Vorteil des erfindungsgemäßen Verfahrens ist, dass durch eine entsprechende Materialzusammenstellung von Matrixmaterial, insbesondere Kunststoffen und Kopplungspartikeln erfindungsgemäße Formkörper hergestellt werden können, die für eine Vielzahl von Anbindungen unterschiedlicher Art geeignet sind. Dabei wird bevorzugt Kunststoffmaterial bzw. das Kunststoffvorläufermaterial als Matrixmaterial eingesetzt.

Bevorzugt ist auch ein erfindungsgemäßes Verfahren, wobei nach dem mechanischen Bearbeiten die Ankopplung von Biomolekülen an von der Kunststoffmatrix wenigstens teilweise unbedeckte Kopplungspartikel erfolgt. Dabei kann das Ankoppeln bevorzugt in verschiedenen Regionen des erfindungsgemäßen Formkörpers durch zueinander verschiedene Biomoleküle erfolgen.

Teil der Erfindung ist auch die Verwendung eines erfindungsgemäßen Formkörpers als Sensor, Biochip, zu Diagnostikzwecken für immunologische Nachweisverfahren, für als Bioreaktor oder Bestandteil eines Bioreaktors, für Labor-auf-dem-Clip-Anwendung, zur Reinigung von Gemischen, die Biomoleküle enthalten, zum Testen von Substanzen auf Verunreinigung, zur Erzeugung einer oder als biokatalytische und/oder bioaktive Oberfläche, zu Zellkulturzwecken, zur Herstellung eines Implantates. Die Verwendung der erfindungsgemäßen Formkörper können aufgrund ihrer Anpassungsmöglichkeiten bei der Herstellung für eine Vielzahl von Verwendungen eingesetzt werden. Dabei handelt es sich um Nachweisverfahren aller Arten, wobei die Kopplungspartikel auf die nachzuweisenden Moleküle abgestimmt werden können bzw. mit Molekülen, wie z.B. Antikörpern beladen werden können, um bestimmte Nachweisreaktionen durchführen zu können. Sie können aber auch zur Reinigung von Gemischen eingesetzt werden, in denen die Kopplungspartikel so ausgestaltet werden, dass sie bestimmte Verunreinigungen aus den Gemischen binden. Letztendlich bestehen für den Fachmann eine Vielzahl von weiteren Einsatzmöglichkeiten der erfindungsgemäßen Formkörper.

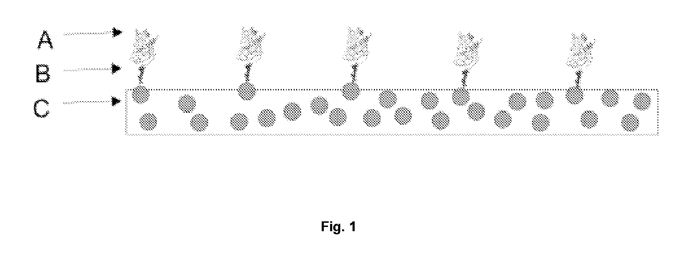

Die Fig. 1 stellt schematisch einen erfindungsgemäßen Formkörper dar, bei dem A ein Biomolekül bezeichnet, das über eine spezifische Gruppe B an ein Kopplungspartikel C gebunden ist. Beispiele für geeignete Kombinationen aus Biomolekül, spezifischer Gruppe und Kopplungspartikel sind in der unten aufgeführten Tabelle 1 dargestellt.

**Tabelle 1**

| A - Biomolekül | B - Spezifische Gruppe | C - Kopplungspartikel |
|---|---|---|
| Proteine (z.B. Antikörper) | Komplexchemie über z.B. Histidin-Tag (His Tag) aber auch über Cystein, Tryptophan oder Phosphatgruppen | Nickel (Ni), Cobalt (Co), Kupfer (Cu) oder Zink (Zn) |
| Peptide | Aminosilane, Epoxysilane, Carboxysilane, mit und ohne Polyethylenglykol (PEG) oder Jeffamin basierten Spacern | Oxide, z. B. Glas (SiO₂), Al₂O₃, ZrO₂, CaCO₃, ITO |
| Nukleinsäuren | Cysteine oder über Dithiodipropionsäure (DPS) | Gold |
| Lipide | Dihydroxyphenylalanin (DOPA) | Eisenpartikel |
| Kohlenhydrate | Spezifische Proteininteraktion (mit z.B. Integrinen) | Magnesium, Kalzium |
| Pharmaka | Carbonsäuren, z.B. in der Acrylsäure (im Acryl) | Acrylpartikel, z. B. PMMA |
| Proteine oder Peptide | Avidin oder Streptavidin | Biotinylierte Polymerpartikel z.B. aus polymilchsäure und deren Derivaten oder Ko-Poymerisaten. |

Weitere Beispiele zur Ankopplung von Biomolekülen an Oberflächen über kovalente chemische Bindungen sind in Willner und Katz (Angew. Chem. 2000, 112, 1230 - 1269) aufgeführt.

Dabei ist dem Fachmann klar, dass die spezifische Gruppe B auch Bestandteil des Biomoleküls sein kann, wie es z.B. beim Histidin-Tag eines Proteins der Fall sein kann.

Der Fachmann wird die Partikelform und die Partikelgröße dem jeweiligen Zweck anpassen. Bevorzugte größte Durchmesser für die Kopplungspartikel liegen im Bereich von 10 nm bis 40 µm, weiter bevorzugt 50 nm bis 10 µm, besonders bevorzugt 100 nm bis 5 µm.

### Beispiel 1 Herstellung von Formkörpern

Für die Ankoppelung von Biomolekülen an Feststoff-Zentren (Kopplungspartikel) wird im ersten Schritt ein Komposit aus dem jeweiligen Feststoff in Pulverform und einem thermoplastischen Polymer hergestellt. Die Herstellung erfolgt in einem temperierbaren Mischer, der den Thermoplasten über die Erweichungstemperatur erwärmt und durch mechanische Verformung plastifiziert. Zur Herstellung der verwendeten Kompositen wurde ein Brabender Plastograph verwendet.

Für die Dispergierung wurde der Thermoplast in den vorgewärmten Mischer gegeben und durch mechanische Scherkräfte plastifiziert. Anschließend werden die Kopplungspartikel als ultrafreines Pulver trocken hinzugegeben. Nach einer Mischzeit von ca. 60 min wurde das Material langsam abgekühlt. Ein Granulat entstand.

Für die Herstellung des Materialkomposites wurden verschiedene Materialkombinationen betrachtet. Als Basiskunststoffe standen Polypropylen (PP), Polystyrol (PS), Polyethylen (PE) und Polyurethan (PUR) zur Verfügung. Ebenfalls wurden die Materialien Polycarbonat (PC) Polymethylmethacrylat (PMMA) verwendet. Als Füllpartikel (Kopplungspartikel) wurden metallische Partikel (Nickel), keramische Partikel (Si02) und Glaskugeln in die aufgeführten Kunststoffe eingebracht.

**Tabelle 2 zeigt eine Übersicht der durchgeführten Versuche:**

| Matrix-material/ Lsg.-mittel | Füllstoff (Kopplungspartikel) | Partikelgröße (mittlerer Durchmesser) [µm] | Bezeichnung | Menge der Kopplungspartikel (an der Gesamtmasse/ dem Gesamtvolumen des Granulats) | Einheit Gesamtmasse |
|---|---|---|---|---|---|
| Lupolen (PE) | Ni | 6,8 | Novamet | 1% | Gew.% |
| Lupolen (PE) | Ni | 2,3 | Fritsch | 1% | Gew.% |
| Lupolen (PE) | Ni | 2,3 | Fritsch | 5% | Gew.% |
| Lupolen (PE) | Ni | 2,3 | Fritsch | 10% | Gew.% |
| PP | Ni | 2,3 | Fritsch | 5 | Gew.% |
| PP | Ni | 2,3 | Fritsch | 10% | Gew.% |
| PS | Ni | 2,3 | Fritsch | 5% | Gew.% |
| PS | Ni | 2,3 | Fritsch | 10% | Gew.% |
| Lupolen (PE) | Glas (S38) | 30 µm | 3M | 5% | Vol% |
| Lupolen (PE) | Glas (S38) | 30 µm | 3M | 10% | Vol% |
| PP | Glas (S38) | 30 µm | 3M | 5% | Vol% |
| PP | Glas (S38) | 30 µm | 3M | 10% | Vol% |
| PS | Glas (S38) | 30 µm | 3M | 10% | Vol% |
| PP | Ohne | | Referenzprobe | 0% | |
| PUR1180A | Glas (S38) | 30 µm | | 10% | Vol% |
| PUR1180A | Ni | 2,3 | Fritsch | 10% | Gew.% |
| PUR685A | Glas (S38) | 30 µm | | 10% | Vol% |
| PUR685A | Ni | 2,3 | Fritsch | 10% | Gew.% |
| Lupolen (PE) | Ni | 2,3 | Novamet | 10% | Gew.% |
| Lupolen (PE) | Aerosil | 14 nm | Plasma-Chem | 1% | Vol% |
| Lupolen (PE) | Aerosil | 14 nm | Plasma-Chem | 5% | Vol% |
| Adisil Rapid | SiO₂ | | Aerosil 200 | 3% | Vol% |
| Adisil Rapid | SiO₂ | | Aerosil 200 | 2,5% | Vol% |

Erläuterungen: Glas (S38) sind Kugel aus Glas von der Firma 3M, Fritsch steht für die Firma Dr. Fritsch GmbH & Co KG.

Mit dem erhaltenen Granulat können nun über konventionelle Spritgussmaschinen Formkörper gefertigt werden. Die Versuchsproben wurden auf einer Laborspritzgussmaschine der Firma "MCP HEK Tooling GmbH" hergestellt.

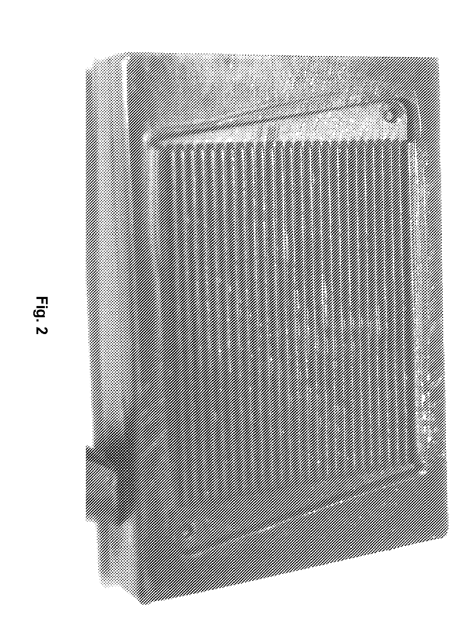

Als Werkzeugform wurden unterschiedliche Geometrien verwendet. Versuche zur Anlagerung an Nickel wurden in der Regel mit dem Formteil "Fotoreaktor" durchgeführt, das in Fig. 2 dargestellt ist.

Für die Geometrie "Fotoreaktor" wurden folgende Materialkombinationen erstellt:

**Tabelle 3: Materialkombinationen Fotoreaktor**

| **Kunststoff** | **Füllmaterial** | **Gehalt des Füllmaterials Ni[Gew%] Glas [Vol%]** | **Temperatur Mischen [°C]** | **Zeit Mischer [min]** | **Temperatur Spritzguß[°C]** |
|---|---|---|---|---|---|
| PS | X | X | 175.00 | 60 | 175,00 |
| PS | Glas | 5 | 175,00 | 60 | 175,00 |
| PS | Glas | 10 | 175,00 | 60 | 175,00 |
| PS | Nickel | 5 | 175,00 | 60 | 175,00 |
| PS | Nickel | 10 | 175,00 | 60 | 175,00 |
| PP | X | X | 170.00 | 60 | ca.180°C |
| PP | Glas | 5 | 170,00 | 60 | ca.180°C |
| PP | Glas | 10 | 170,00 | 60 | ca.180°C |
| PP | Nickel | 5 | 170,00 | 60 | ca.180°C |
| PP | Nickel | 10 | 170,00 | 60 | ca.180°C |
| PE | X | X | 120 | 60 | 135,00 |
| PE | Glas | 5 | 120 | 60 | 135,00 |
| PE Glas | Glas | 10 | 120 | 60 | 135,00 |
| PE Nickel | Nickel | 5 | 120,00 | 60 | 135,00 |
| PE | Nickel | 10 | 120,00 | X | 135,00 |
| PMMA | X | X | 220,00 | X | 220,00 |
| PUR 1180 | X | X | 210,00 | 60 | 185,00 |
| PUR 1180 Arburg | X | X | X | 60 | 210,00 |
| PUR 685 | X | X | X | X | 185,00 |
| PUR685 | X | X | X | X | 185,00 |
| PUR685 Arburg | X | X | X | X | 205,00 |

### Beispiel 2 Formkörper

Als weitere Geometrie wurden im Spritzgussverfahren "Sticksel" (kugelig-zylindrische Formkörper) gefertigt. Tabelle 4 zeigt eine Übersicht der gefertigten Materialkombinationen:

**Tabelle 4: Materialkombination Sticksel**

| Kunststoff | Kopplungspartikel | Gehalt der Kopplungspartikel [Vol%] | Temperatur Mischen [°C] | Zeit Mischer [min] | Temperatur Spritzguß | Luftdruck [bar] | Nachdruck bei Einstellung 30 sec |
|---|---|---|---|---|---|---|---|
| PE | X | X | 125 | 15 | 125 | 4 | 0,5 |
| PE | X | X | X | X | 125 | 4 | 0,5 |
| PE | Glas (S38) | 10 | 125 | 15 | 128-130 | 4 | 0,5 |
| PE | Nickel (Novam et) | 10 | 125 | 15 | 128-130 | 4 | 0,5 |

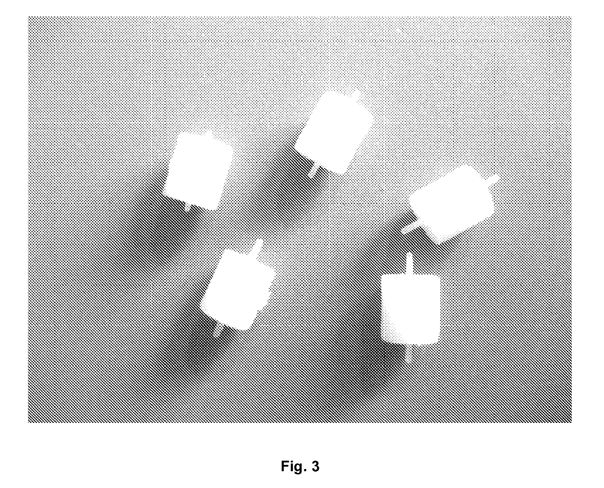

Fig. 3 zeigt abgeformte Spritzgussteile (Formkörper) mit zehn Volumenprozent Glaskugeln (mittleren Teilchendurchmesser von 20 µm) in einer Polyethylen-Matrix.

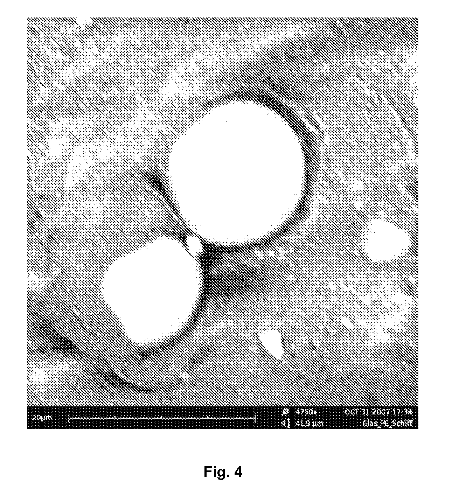

Für die biochemische Reaktion (Kopplung) müssen nun noch die aktiven Zentren (GlasKugeln) freigelegt werden. Dies kann über verschiedene Verfahren, wie z.B. chemisches oder plasmachemisches Ätzen erfolgen. Bei diesen Verfahren werden jedoch häufig neue unselektive aktive Zentren in Form von Carboxly- oder ähnlichen Gruppen an der Oberfläche der Polymere aktiviert, die ein ankoppeln von Biomolekülen auf der gesamten Polymeroberfläche begünstigen. Um diese Oberflächenaktivierung zu vermeiden wurde eine mechanische Abtragsmethode für das Freilegen der Partikel an der Kompositoberfläche gewählt. Die Kompositproben wurden jeweils ca. 5 Minuten auf einer Schleif- und Poliermaschine des Typs Stuers LaboPol-21 mit Körnung zwischen P500 und P1200 abgeschliffen. Nach der Behandlung liegen die Füllstoffpartikel offen an der Oberfläche, wie in Fig. 4 zu erkennen ist.

Fig. 4 zeigt die Oberfläche eines Formkörpers aus zehn Volumenprozent Glaskugeln, die in eine Polyethylen-Matrix eingebracht worden sind. Deutlich zu erkennen sind zwei teilweise freiliegende Glaskugeln mit einem Durchmesser von kleiner 15 µm.

### Anwendungsbeispiel 1: Polyethylenstäbe mit silanisierten ITO-Partikeln

Die kovalente Ankopplung von Biomolekülen an fixierte Partikel in Polymeren bietet die Möglichkeit, einen biochemischen Sensor zu erstellen. Hierfür ist jedoch noch eine Auswertung bzw. Erkennung des Ankopplungsvorganges notwendig. Eine sehr einfache und gleichzeitig effektive Möglichkeit bietet die elektrische Auswertung von Oberflächenveränderungen oder Oberflächenpotentialen. Eine notwendige Rahmenbedingung hierfür ist eine hinreichend hohe elektrische Leitfähigkeit der Probe. Da die Ankopplung der Biomoleküle an die eingebrachten Kopplungspartikel geschehen soll, sollten diese Partikel eine hinreichende elektrische Leitfähigkeit zeigen. Als Kopplungsmechanismus wurde die Anbindung eines Silans an eine Oxidschicht gewählt. Die hier aufgezeigten notwendigen Eigenschaften verbinden halbleitende Oxide, wie z.B. das Indiumzinnoxid ITO. Durch die Kombination solcher Oxide in einem elektrisch nicht leitenden Polymer wie beispielsweise Polyethylen ergibt sich die Möglichkeit, eine kovalente Ankopplung von Biomolekülen an die aktiven Oxid-Partikel über die Veränderung von elektrischen Kenngrößen zu messen. Für die Übertragung der elektrischen Signale von der Oberfläche des Komposits bis zur Auswerteelektronik ist eine elektrische Leitfähigkeit des Komposits notwendig. Dies kann durch die Variation des Füllgehalts erreicht werden. Bei Konzentrationen im Bereich von ca. 20 bis 30 Volumenprozent kommt es in solchen Kompositen zur Perkolation. Ab dieser Konzentration kommt es durch statistische Verteilung der Partikel in der Matrix zu zusammenhängende Gebieten (Cluster) aus Partikeln, die über Berührungspunkte untereinander verbunden sind. Über diese Cluster (aus elektrisch halbleitenden Partikeln) kann die elektrische Information durch das gesamte Komposit übertragen werden. Die notwendige Konzentration zur Perkolation ist von der Partikel-Geometrie, dem Material und weiteren Faktoren abhängig, so dass die notwendige Konzentration für jedes System erneut ermittelt werden muss. Mit diesem elektrisch leitfähigen Komposit können nun die zu untersuchenden Reaktionsmechanismen elektrisch vermessen werden. Dies kann beispielsweise über eine Impedanzmessung oder die Cyclo-Voltametrie erfolgen. Gleichzeitig verhindert die nur extrem geringe Leitfähigkeit des reinen Polymers die Aufzeichnung von nicht gewünschten Kopplungen zwischen Biomolekülen und der Polymeroberfläche.

Um dieses Anwendungsbeispiel zu testen, wurde in ein PE mit 30 vol% ITO Pulver vermischt. Das so entstandene Kompositmaterial wurde auf einem Miniextruder zu flachen Stäben verarbeitet. Die Versuche zur Ankopplung zeigten (vgl. Anwendungsbeispiel 2), dass bei den mit Aminosilan und dem Biomolekül CF-Ala (Carboxidfluorescin-Alanin) behandelten Formkörpern im Fluoreszenzmikroskop ein Signal zu sehen war. Die Kontrolle ohne Aminosilan, aber mit Biomolekül CF-Ala" zeigte kein Signal, da die Kopplungspartikel nicht mit einer reaktiven Funktion ausgestattet worden waren.

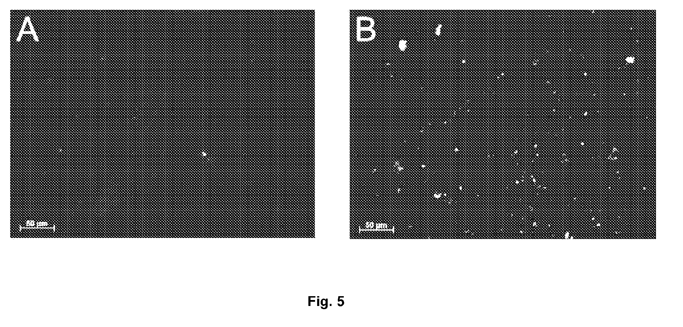

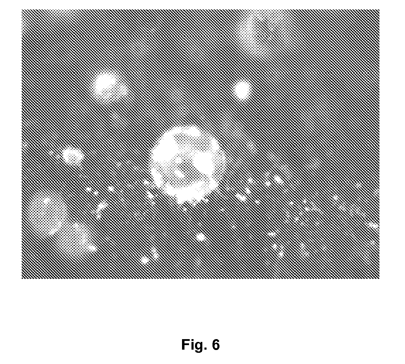

Die Figur 5 zeigt Fluoreszensmikroskopaufnahmen von Polyethylen Formkörpern, die gemäß dem Anwendungsbeispiel 1 hergestellt wurden. Dabei wurde bei dem Formkörper, der in Figur 5 A dargestellt ist, auf eine Silan-Funktionalisierung verzichtet. Der in Figur 5 B dargestellte Formkörper umfasst dagegen mit Aminosilan funktionalisierte Partikel. Es lässt sich deutlich erkennen, dass an dem Formkörper der Abbildung A keine Ankopplung des Biomoleküls CF-Ala stattfand, während im Bild B deutlich Signale der angekoppelten Biomoleküle zu erkennen sind. Aufnahmen wurden mit einem Fluoreszenzmikroskop (Axio Imager.M1 von Zeiss) erstellt und nachfolgend in SW konvertiert.

### Anwendungsbeispiel 2: Ankopplung von Carboxidfluorescin-Alanin an Formkörpern

Mit dem Festphasenpeptid-Synthesiser wurde ein Carboxidfluorescin-Molekül mit vier Alaninmolekülen gekoppelt. Dieses gekoppelte Molekül (CF-Ala) sollte als Probe für ein Biomolekül an erfindungsgemäße Formkörper gekoppelt werden. Dazu wurden als Formkörper die Sticksel aus Beispiel 2 verwendet, und zwar in der in der Tabelle 4 beschriebenen Materialkombination aus Polyethylen mit 10 Vol% Glas (S 38) als Kopplungspartikel. Als Kontrolle wurden Sticksel aus PE eingesetzt, in denen keine Kopplungspartikel enthalten waren.

Als nächster Schritt wurden alle Sticksel einer Silanisierung mit Aminosilan unterzogen. Dazu wurden die Sticksel mit einer 2 %igen (v/v) Lösung vom 3-Aminopropyltrimethoxysilan (APTMS) in Aceton behandelt. Die Silanisierung erfolgte für 30 Sekunden. Nach erfolgter Inkubation würde die Silanisierungslösung abgesaugt und die Sticksel Formkörper mit 100% Aceton gewaschen und anschließend mit Stickstoff getrocknet. Die Kopplung des CF- Ala an die Sticksel wurde mit 1-Ethyl-3-(3-dimethylaminopropyl)-carbodiimid (EDC, 10 mg/ mL in 2-(N-Morpholino)ethansulfonsäure

(MES) Puffer) als Crosslinker und N-Hydroxysuccinimid (NHS, 0,6 mg/mL in MES Puffer), als Stabilisator des Zwischenproduktes aus durch EDC aktivierten CF- Ala ( 1 mg/ mL in MES Puffer) durchgeführt. Die Inkubation erfolgte für 2 Stunden bei Raumtemperatur auf einem Orbitalschüttler. Nachfolgend wurden die Sticksel Formkörper mit Phosphat gepufferterter Salzlösung mit Tween 100 (PBS-T) für eine Stunde gewaschen, getrocknet und unter einem Fluoreszenmikroskop betrachtet (Carl Zeiss Axio Imager M1, Filtersatz F9, Belichtungszeit 15 Millisekunden).

Die Figur 5 stellt eine fluoreszenzmikroskopische Abbildung der Formkörper gemäß Anwendungsbeispiel 2 dar. Die Abbildung wurde erstellt nach Kopplung von CF-Alanin an die Kopplungspartikel. Dabei sind die eingebetteten Partikel deutlich aufgrund der erfolgten Biofunktionalisierung (Kopplung von CF-Alanin) im Fluoreszenzmodus des Mikroskops zu erkennen.

Dabei wurden die Oberflächen der Sticksel (die vor der Silanisierung angeschliffen worden waren) einer Lösung von CF- Ala in Ethanol für 15 Minuten und 30 Minuten ausgesetzt. Als Ergebnis ließ sich eine deutlich erhöhte Ankopplungskonzentration von CF-Ala auf den mit Kopplungspartikeln versehenen Sticksel nachweisen. Die Konzentrationserhöhung der gekoppelten Biomoleküle basierte dabei auf einer stark erhöhten Moleküldichte im Bereich der Kopplungspartikel.

### Anwendungsbeispiel 3: Kopplung an Nickelkopplunqspartikel

Gemäß Beispiel 1 hergestellte Formkörper vom Typ Fotoreaktor aus Polyethylen (Lupolen) als Matrixmaterial und 5 Gew.% Nickelpartikel mit einem mittleren Partikeldurchmesser von 2,3 µm (Fritsch) als Kopplungspartikel wurden einer Lösung des Proteins TNFalpha, das über einen Histidin-Tag verfügt, ausgesetzt. Als Kontrolle diente das eingesetzte Matrixmaterial ohne Nickelpartikel. Die Formkörper wurden jeweils mittels mechanischem Abtrag durch Schleifen. Die Formkörper mit und ohne Nickelpartikel wurden mit einer TNFalpha Lösung von 10 mg/ mL in Phosphat gepufferter Salzlösung (PBS) für 3 Stunden bei Raumtemperatur auf einem Kippschüttler inkubiert. Anschließend wurde überschüssiges bzw. nicht gebundenes Protein mit einer Waschlösung (PBS mit 0,1 % [v/v] des Detergenzes Tween 100) behandelt. Das gebundene Protein TNFalpha wurde anschließend durch einen antikörpervermittelten Enzymtest nachgewiesen, wie dieser auch dem Stand der Technik bekannten Enzym gebundenen Sandwich Essay (ELISA) durchgeführt wird. Hierbei wurde das TNFalpha mit einem anti-TNF Antiköper, der mit dem Enzym Peroxidase gekoppelt war, detektiert. Die Detektion erfolgte durch die Zugabe der Substanz Tetramethylbenzidin (TMB). Das TMB wird von einer farblosen Substanz durch die Peroxidase in einen blauen Farbstoff umgewandelt. Dabei konnte mittels des enzymgekoppelten Antikörpernachweises gezeigt werden, dass eine Anlagerung der His-Tag gebundenen Proteine fast ausschließlich an den Nickelpartikeln des mit Nickel gefüllten Formkörpers erfolgte. Auf dem Kontrollformkörper wurde nur eine sehr schwache unspezifische Anbindung beobachtet.

## Patentansprüche

1. Formkörper, umfassend eine Matrix aus einem Material, ausgewählt aus der Gruppe bestehend aus Metall, Keramik und polymerem Kunststoff, und in die Matrix eingebettete Kopplungspartikel, wobei ein Teil der Oberfläche des Formkörpers in einer geometrischen Form oder einem regelmäßigen Muster und/oder eine Fläche des Formkörpers vollständig oder die gesamte Oberfläche des Formkörpers mechanisch bearbeitet ist.

2. Formkörper, nach Anspruch 1, wobei die Matrix eine polymere Kunststoffmatrix ist und der Formkörper mechanisch bearbeitet ist, so dass ein Teil der Partikel wenigstens teilweise von der Kunststoffmatrix unbedeckt ist und/oder wobei das mechanische Bearbeiten erfolgt ist durch Schleifen, Fräsen und/oder Sandstrahlen.

3. Formkörper nach einem der vorangehenden Ansprüche, wobei der Formkörper undurchlässig für Wasser ist.

4. Formkörper nach einem der vorangehenden Ansprüche, wobei wenigstens 1 % einer Fläche des Formkörpers mechanisch bearbeitet ist.

5. Formkörper nach einem der vorangehenden Ansprüche, wobei der Formkörper so mechanisch bearbeitet ist, dass ein Teil der Partikel aus der Oberflächenebene des Formkörpers herausragt und teilweise freiliegt.

6. Formkörper nach einem der vorangehenden Ansprüche, wobei die Kunststoffmatrix Material umfasst, ausgewählt aus der Gruppe bestehend aus Thermoplasten, Duroplasten und Elastomeren und/oder wobei das Matrixmaterial ausgewählt ist aus der Gruppe bestehend aus Polypropylen PP, Polystyrol PS, Polyurethan PU, Polycarbonat PC, Polymethylmethacrylat PMMA, Polyoxymethylen POM, Polyvenylchlorid PVC, Polyethylen PE, Thermoplastischer Polyurethan TPU, Polyetheretherketon PEEK, Polytetrafluorethylen PTFE und Biopolymeren, insbesondere thermoplastische Stärke und Polymilchsäure.

7. Formkörper nach einem der vorangehenden Ansprüche, wobei die Kopplungspartikel ein Material umfassen oder aus einem Material bestehen, ausgewählt aus der Gruppe bestehend aus Metall, Metalloxid, Keramik, Glas und Kunststoffen mit höherer Schmelztemperatur als das Matrixmaterial und/oder wobei die Kopplungspartikel zumindest im Bereich, wo sie von der Matrix unbedeckt sind, wenigstens teilweise zur Verbesserung der Kopplungseigenschaften an Biomoleküle oberflächenmodifiziert sind und bevorzugt die Oberflächenmodifizierung die Einführung funktioneller Gruppen umfasst oder daraus besteht.

8. Formkörper nach einem der vorangehenden Ansprüche, herstellbar oder hergestellt unter Einsatz eines oder durch ein Spritzgussverfahren, Extrusion, Heißprägen, Prägen oder Formgießen und nachfolgendes mechanisches Bearbeiten.

9. Formkörper nach einem der vorangehenden Ansprüche, umfassend an seiner Oberfläche angekoppelte Biomoleküle und wobei die Biomoleküle bevorzugt ausgewählt sind aus der Gruppe bestehend aus Proteinen, Peptiden, Kohlenhydraten, Lipiden, Kohlenhydraten, Nukleinsäuren, Hormonen, Aminosäuren und Nukleotiden.

10. Formkörper nach Anspruch 9, wobei die Biomoleküle zu ≥ 1%, bevorzugt ≥ 85%, weiter bevorzugt ≥ 95% besonders bevorzugt ≥ 99% an die Kopplungspartikel gekoppelt sind.

11. Formkörper nach Anspruch 9 oder 10, wobei die Biomoleküle über Komplexierung oder kovalent angekoppelt sind.

12. Verfahren zum Herstellen eines Formkörpers nach einem der Ansprüche 1 bis 11, umfassend die Schritte:
a) Bereitstellen eines Kunststoffmaterials und/oder eines Kunststoffvorläufermaterials und/oder eines Metallmaterials und/oder eines keramischen Vorläufermaterials,
b) Bereitstellen von Kopplungspartikeln,
c) Mischen der Kopplungspartikel mit dem Kunststoffmaterial und/oder dem Kunststoffvorläufermaterial und/oder dem Metallmaterial und/oder dem keramischen Vorläufermaterial,
d) Formen eines Formkörpers aus der Mischung und
e) mechanisches Bearbeiten des Formkörpers, so dass ein Teil der Oberfläche des Formkörpers in einer geometrischen Form oder einem regelmäßigen Muster und/oder eine Fläche des Formkörpers vollständig oder die gesamte Oberfläche des Formkörpers mechanisch bearbeitet ist.

13. Verfahren nach Anspruch 12, wobei im Schritt a) ein Kunststoffmaterial und/oder ein Kunststoffvorläufermaterial bereitgestellt wird und der Schritt d) so erfolgt, dass ein Teil der Partikel wenigstens teilweise von der Kunststoffmatrix unbedeckt ist.

14. Verfahren nach Anspruch 12 oder 13, wobei nach dem mechanischen Bearbeiten eine Ankopplung von Biomolekülen an von der Matrix wenigstens teilweise unbedeckte Kopplungspartikel erfolgt.

15. Verwendung eines Formkörpers nach einem der Ansprüche 1 bis 11 als Sensor, Biochip, zu Diagnostikzwecken für immunologische Nachweisverfahren, als Bioreaktor oder Bestandteil eines Bioreaktors, für Labor-auf-dem-Clip-Anwendung, zur Reinigung von Gemischen, die Biomoleküle enthalten, zum Testen von Substanzen auf Verunreinigung, zur Erzeugung einer oder als biokatalytische und/oder bioaktive Oberfläche, zu Zellkulturzwecken, für zellbiologische und immonologische Untersuchungen auf biofunktionalisierten Formkörpern, zur Herstellung eines Implantates oder eines biofunktionalisierten Medizinproduktes.
